(19) Europäisches Patentamt / European Patent Office / Office européen des brevets

(11) **EP 2 604 191 A1**

(12) **EUROPEAN PATENT APPLICATION**

(43) Date of publication:
**19.06.2013 Bulletin 2013/25**

(51) Int Cl.:
*A61B 17/00* (2006.01)  *A61B 17/34* (2006.01)

(21) Application number: **11193758.7**

(22) Date of filing: **15.12.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **University College Cork
Cork City (IE)**

(72) Inventor: **The designation of the inventor has not
yet been filed**

(74) Representative: **Purdylucey Intellectual Property
6-7 Harcourt Terrace
Dublin 2 (IE)**

(54) **A magnetic coupling to improve placement of gastroenteral feeding tubes and colostomy tubes**

(57) The invention provides a kit suitable for performing a surgical procedure that involves a percutaneous incision and placement of a tube within the incision connecting the inside of the body with the outside. Examples of such procedures include a percutaneous endoscopic gastrostomy, percutaneous endoscopic jejunonstomy and a sigmoid colostomy. The kit comprises a tube and a first magnet that in use couples with a second magnet across a gastrointestinal and abdominal wall, in which the first magnet is located inside the gastrointestinal tract and the second magnet is located externally of the abdomen. The first magnet includes a through-hole dimensioned for receipt of the tube, wherein the through-hole of the first magnet is capable of aligning with a through-hole of the second magnet upon coupling of the magnets to provide a guide path for passage of the tube.

Figure 2

**EP 2 604 191 A1**

**Description**

**Field of the Invention**

The invention relates to a method and kit for placement of a percutaneous tube. In particular, the invention relates to a method and kit for use in placement of a percutaneous tube in percutaneous endoscopic gastrostomy and percutaneous endoscopic colostomy.

**Background to the Invention**

Enteral nutrition is a means of delivering nutrition to patients who would otherwise be unable to feed themselves for a variety of reasons: neurological impairment, dysphasia (difficulty in swallowing) after surgery, oral cavity tumours, anorexia, or as a preventative for aspiration pneumonia. There are a number of types of enteral feeding solutions used but the most common are nasogastric tubes (NGT) and gastroenteral tubes, placed using the percutaneous endoscopic gastrostomy (PEG) technique. While NGT nutrition is often preferred by radiologists, PEG is the preferred technique amongst gastroenterologists, endoscopists and surgeons. This is because PEG tubes are easier to tolerate, show better nutritional results and patients with PEG tubes have higher survival rates than those with NGT tubes, even when PEG tube patients are in more advanced stages of illness.

In some palliative scenarios, a modified PEG technique will employ placement of the feeding tube into the first portion of small intestine, the jejunum. This percutaneous endoscopic jejunostomy (PEJ) technique is equally relevant to the current invention.

However, PEG tube placement is not without complications including gastric perforation, tube blockage, site infection, PEG tube dislocation and inadvertent puncture of peripheral organs such as the colon that can become sandwiched in between the gastric and abdominal walls during the placement of the PEG tube.

Percutaneous endoscopic sigmoid colostomy (PEC) is a variation of the percutaneous endoscopic gastrostomy technique. PEC offers an alternative treatment for patients who have tried conventional treatment options without success. Various surgical techniques as an alternative to PEC include sigmoidopexy, sigmoidoplasty, trephine stoma to resection with primary anastomosis. Traditional treatment options for sigmoid volvulus comprise endoscopic decompression and/or open resection. However, these treatment options have varying success with endoscopic decompression having a recurrence rate of approximately 40% and open resection may be contraindicated for frail, elderly patients or the severely immunocompromised.

It is an object of the present invention to overcome at least some of the above-described problems.

**Summary of the Invention**

According to the present invention there is provided, as set out in the appended claims, a kit suitable for performing a percutaneous endoscopic gastrostomy or a similar procedure and comprising , a feeding tube, optionally a guidewire, and a first magnet capable of coupling with a second magnet across a gastrointestinal (i.e. stomach, small intestine or colon) and abdominal wall, the first magnet including a through-hole dimensioned for receipt of the feeding tube, wherein the through-hole of the first magnet is capable of aligning with a through-hole of the second magnet upon coupling of the magnets to provide a guide path for passage of the guidewire and/or tube. Preferably, the kit includes two magnets, namely the first magnet (hereafter referred to as "internal" magnet") and a second magnet (hereafter referred to as "external" magnet) capable of coupling together across a gastrointestinal wall and abdominal wall, each magnet including a through-hole dimensioned for receipt of the tube. Alternatively, the kit may include just a single magnet (internal magnet), and may be used with an external magnet such as a reusableelectromagnet or permanent electromagnet, which may or may not form part of the kit.

The present invention provides a magnetic coupling (*i.e.,* two opposing north/south magnetic surfaces) consisting of two magnets which are coupled across a gastrointestinal (i.e. gastric, intestinal or colon) and abdominal walls to provide a guide path for making an incision through the abdominal and gastrointestinal walls, and for subsequent passage of a guide wire and/or tube. Use of the magnetic coupling ensures that the incision is made with accuracy, and does not stray away from the target tissue. Further, since coupling of the two rings will only occur within a given distance, this technique may be used to improve tube placement by preventing coupling where part of an organ is sandwiched between the two magnets. In other words, the magnets can be chosen such that they will couple across a gastric, intestine or colon and an abdominal wall, but not couple when an organ is inadvertently sandwiched between the gastric and abdominal walls.

The kit of the invention is particularly suitable for performing percutaneous endoscopic gastrostomy, in which the magnets couple across a gastric and abdominal wall. However, the kit of the invention may also be employed to perform other procedures involving percutaneous incisions, for example a colostomy procedure, specifically a sigmoid colostomy, in which the magnets couple across a colon wall and abdominal wall.

The or each magnet includes a through hole which is dimensioned to receive a feeding tube in the case of a PEG procedure and a colostomy tube in the case of a colostomy procedure. Typically, the or each magnet has an annular shape, and ideally is a ring-shaped magnet, although other shapes of magnet may be employed provided that they incorporate a hole which can be employed to guide the percutaneous incision and subsequent insertion of medical devices such as a feeding tube or guide wire. The or each magnet may comprise a ferromagnetic or ferrimagentic material, or may comprise a paramagnetic material. The or each magnet may further comprise an electromagnet with active current-carrying coils or a permanent electromagnet with permanent magnetic core and 'turn-on' coils.

In one embodiment of the invention, the kit includes a scoping device suitable for positioning the internal magnet inside the gastrointestinal tract (i.e. the stomach) and having a distal end, a proximal end, and means for engaging the internal magnet disposed on the distal end. Suitably, the means for engaging the internal magnet comprises an inflatable balloon, wherein inflation of the balloon secures the magnet at the distal end of the device and deflation of the balloon releases the magnet from the distal end of the device. Other means for engaging the internal magnet include, for example, use of a magnet on the distal end of the scope suitable for coupling with the internal magnet. The scoping device may be selected from an endoscope or a bronchoscope.

Preferably, the external magnet includes an indicator for confirming when the two magnets have coupled together. The indicator may be a visual or audio indicator. Ideally, it is a visual indicator, for example a light emitting diode which lights to confirm when the magnets have coupled. The light may be actuated by a sensor which is capable of detecting when coupling of the magnets has taken place.

Typically, the or each magnet is selected from the group comprising neodymium-iron-boron, soft or stainless steel, iron, cobalt, and nickel. One preferred embodiment employs both magnets are N52 grade neodymium-iron-boron.

Preferably, the kit includes one, more, or all of a syringe, a cannula, a retrieval snare, a bolus adapter, and a retention ring.

In another aspect, the invention relates to an assembled magnetic coupling suitable for assisting placement of a gastroenteralfeeding tube, jejunoenteral feeding tube or a colostomy tube and comprising an internal magnet disposed inside the stomach or colon of an individual, and bearing against an inside of the stomach, intestine or colon wall, magnetically coupled to an external magnet disposed externally against the abdomen of the individual, each magnet comprising a through-hole dimensioned for receipt of the tube, wherein the through-holes are aligned to provide a path for passage of the tube through the abdominal and stomach, or colon, walls.

Typically, the internal and external magnets are ring-shaped magnets.

Alternatively, the external magnet is an electromagnet or electropermanent magnet.

Preferably, the external magnet includes an indicator for confirming when the two magnets have coupled together. The indicator may be a visual or audio indicator. Ideally, it is a visual indicator, for example a light emitting diode which lights to confirm when the magnets have coupled. The light may be actuated by a sensor which is capable of detecting when coupling of the magnets has taken place. One embodiment for the external magnet's indicator is a Hall-effect magnetic field sensor. A second embodiment is a modified Reed switch.

In a further aspect, the invention provides a scoping device suitable for being inserted into the body and relaying images from inside the body to a viewing device located outside the body, the device having a distal end and a proximal end, characterised in that the distal end of the device comprises an inflatable balloon, typically in the form of a collar, wherein inflation and deflation of the balloon can be controlled from the proximal end of the device.

Typically, the scoping device is selected from an endoscope, colonoscope or a bronchoscope.

In a further aspect, the invention relates to a kit suitable for providing a percutaneous incision useful for receipt of a percutaneous tube, for example a gastroenteral or jejunoenteral feeding tube or a colostomy tube, the kit comprising a scoping device of the invention, and an internal magnet having a through hole, wherein the through-hole is dimensioned for receipt of the inflatable balloon when deflated but not when inflated. Preferably, the kit comprises two magnets adapted to couple together across an abdominal wall and a gastric or colon wall, each magnet having a through hole dimensioned to align when the magnets couple to provide a guide path for the percutaneous incision.

The invention also relates to a method of providing a percutaneous incision in an individual suitable for receipt of a percutaneous tube, for example a gastroenteral or jejunoenteral feeding tube or a colostomy tube, which method employs a pair of magnets capable of coupling together across the intestinal and abdominal walls, each magnet having a through hole dimensioned for receipt of the tube and capable of aligning to provide a guide path for the percutaneous incision, the method comprising the steps of:

- positioning an internal magnet inside the stomach, intestine or colon of the individual adjacent the stomach, intestine or colon wall at a position where the tube is to be located;
- positioning an external magnet on the external abdomen close to the first magnet;
- allowing the two magnets couple together across the stomach and abdominal, or intestine and abdominal, or colon and abdominal, walls to provide a guide path for the incision; and

- employing a piercing device to make an incision through the abdominal and stomach, or abdominal and intestine, or abdominal or colon, walls along the guide path.

Typically, the piercing means is a syringe. Suitably, an initial incision is made prior to insertion of a cannula into the incision thus formed.

In this specification, the term "magnet" should be understood to include permanently magnetised materials, such a ferromagnetic and ferromagnetic materials, and paramagnetic materials which are only weakly attracted to magnets. The magnet may be selected from the group comprising high grade (e.g., N52 grade) neodymium-iron-boron, stainless steel, iron, cobalt, and nickel. In the specification, the term "electromagnet" should be understood to mean a type of magnet in which the magnetic field is generated by an electric current. The term "permanent electromagnet" should be understood to mean a type of magnet in which the magnetic field is generated by pairs of permanent magnetic rods which are capable of being turned 'on' or 'off' by means of a pulse demagnetising magnetic field in a coil.

In the specification, the term "gastric magnet" should be understood to mean any magnet which can be used within an endoscope, gastroscope, or bronchoscope and that the term is interchangeable with the terms "first magnet" and "internal magnet".

In the specification, the term "jejunal magnet" should be understood to mean any magnet which can be used within an endoscope, gastroscope, or bronchoscope and that the term is interchangeable with the terms "first magnet" and "internal magnet".

In the specification, the term "abdominal wall magnet" should be understood to mean any magnet which can be used on the abdominal wall external a patient and that the term is interchangeable with the terms "second magnet" and "external magnet".

In this specification, the term "percutaneous incision" should be understood to mean an incision through the abdominal wall and a wall of the gastrointestinal tract, for example the stomach or colon wall.

The term "percutaneous tube" should be understood to mean a tube that is intended to cross the abdominal and gastrointestinal wall, for example a gastroenteral or jejunoenteral feeding tube, or a colostomy tube.

The term "gastrointestinal wall" should be understood to mean a wall of a part of the gastrointestinal tract, and includes gastric walls and colon walls.

In this specification, the term "scoping device" should be understood to mean an imaging device that is inserted into an individual orally or rectally and used to relay images from inside the patient to a viewing device located externally of the patient. Examples of scoping devices include endoscopes and bronchoscopes. They generally employ a CCD camera or a fibre optic camera.

**Brief Description of the Drawings**

The invention will be more clearly understood from the following description of an embodiment thereof, given by way of example only, with reference to the accompanying drawings, in which:-

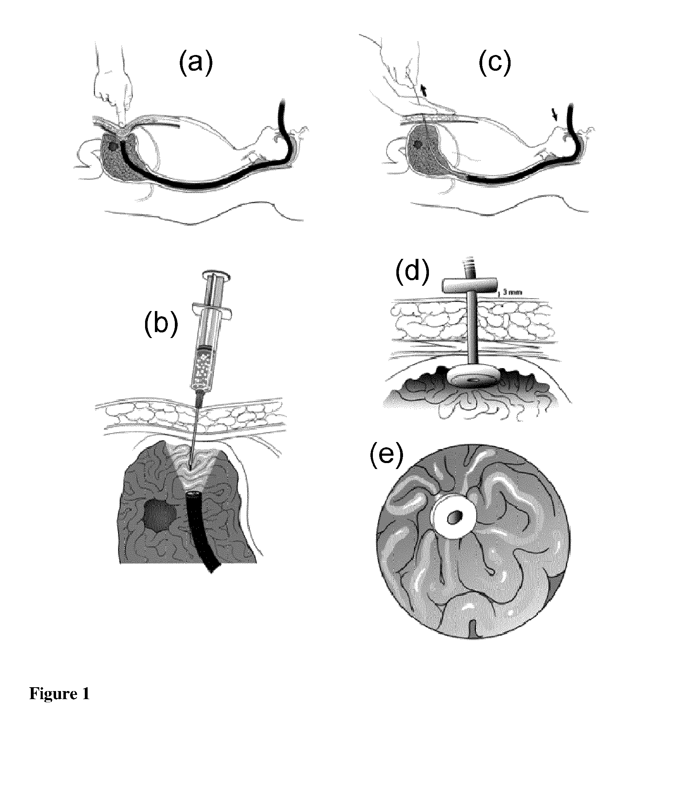

Figure 1 illustrates an outline of the current PEG tube placement procedure involving (a) endoscopic trans-illumination, (b) transabdominal needle perforation, (c) guidewire-introduced tube placement, (d) mechanical interlocking and (e) fixation (reproduced from Ponsky 2004 (Percutaneous endoscopic gastrostomy. Journal of Gastrointestinal Surgery; 8(7):901-904));

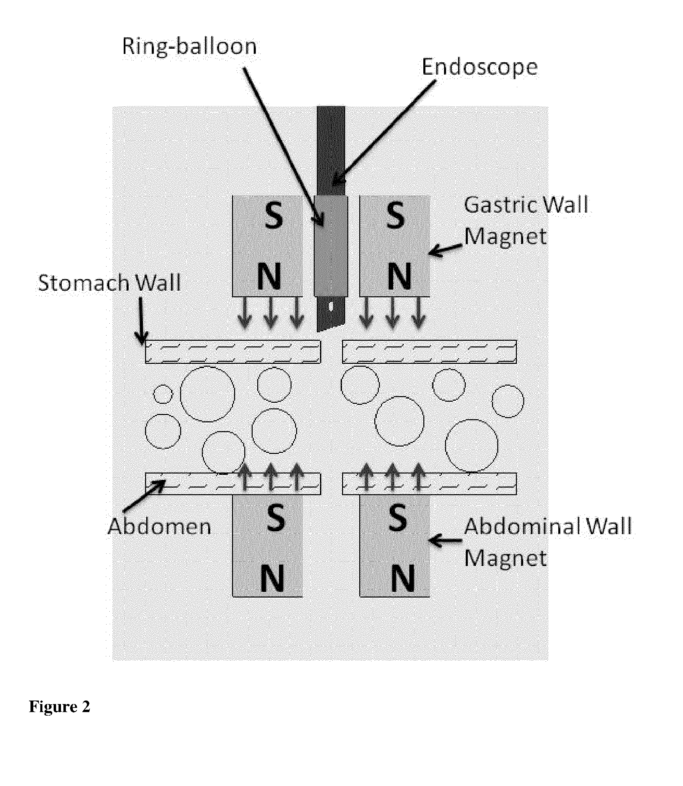

Figure 2 illustrates a magnetic coupling of the present invention to aid in PEG tube placement;

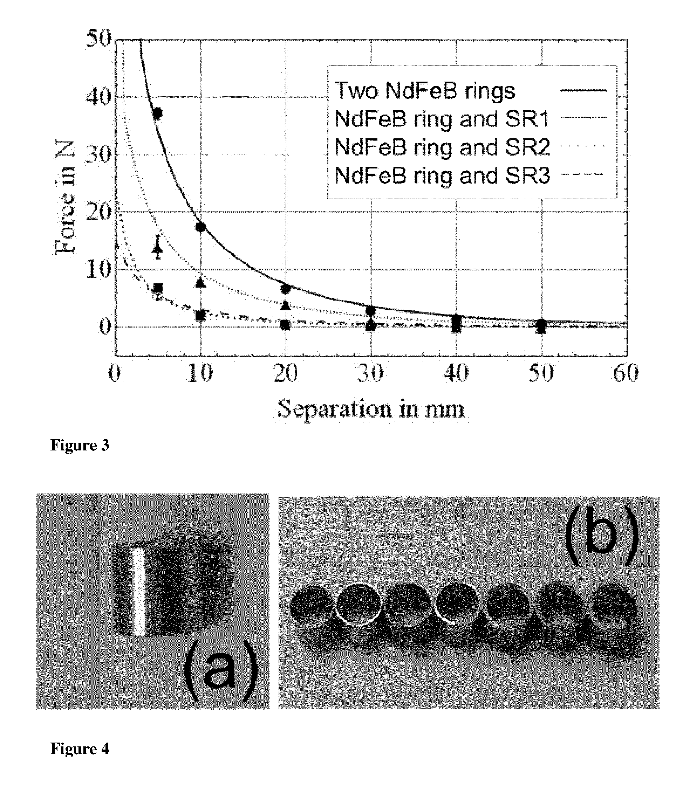

Figure 3 illustrates a graph demonstrating force-separation characteristics between an N52 grade ring magnet and (i) a second identical magnetic ring (MR), and (ii) three stainless steel rings of varying thicknesses and lengths; SR1 with 26mm OD, 20mm ID and 25mm H, SR2 with 26mm OD, 20mm ID and 12.5mm H and SR3 with 22mm OD, 20mm ID and 12.5mm H. Gravitational forces are ignored;

Figure 4 illustrates a gold-plated N52 grade neodymium-iron-boron ring (a) and stainless steel rings of various thicknesses (b) which were used in testing of the present invention;

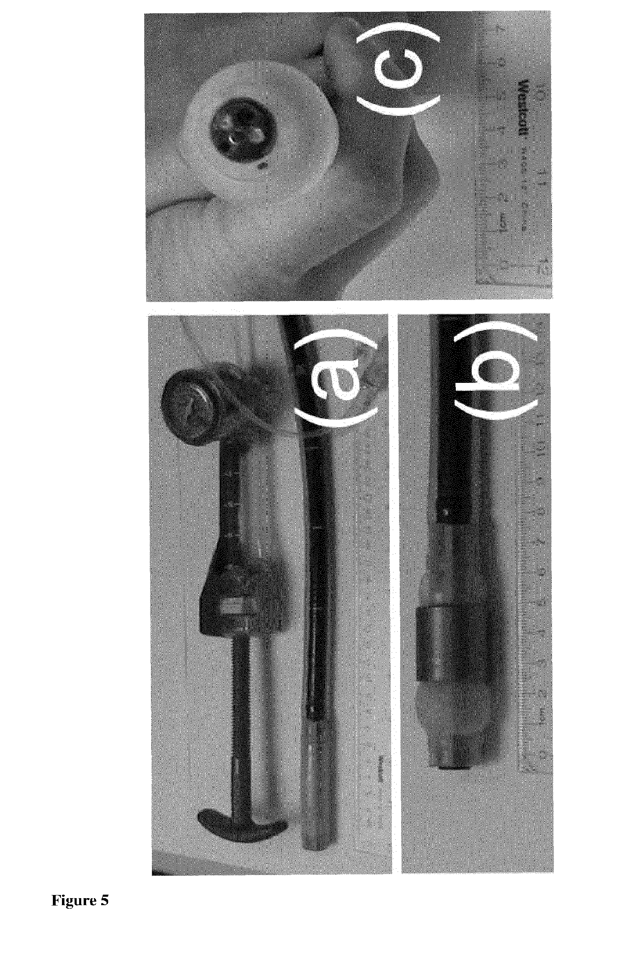

Figure 5 illustrates a modified PEG kit for use with the present invention where (a) a modified endotraceal tube was used as the ring balloon, fitting snugly over the 12mm endoscope. (b) When inflated, the balloon fixed the ring on the endoscope's shaft. (c) The balloon also minimised risk of tissue tearing due to the magnet's edges upon insertion of the endoscope; and

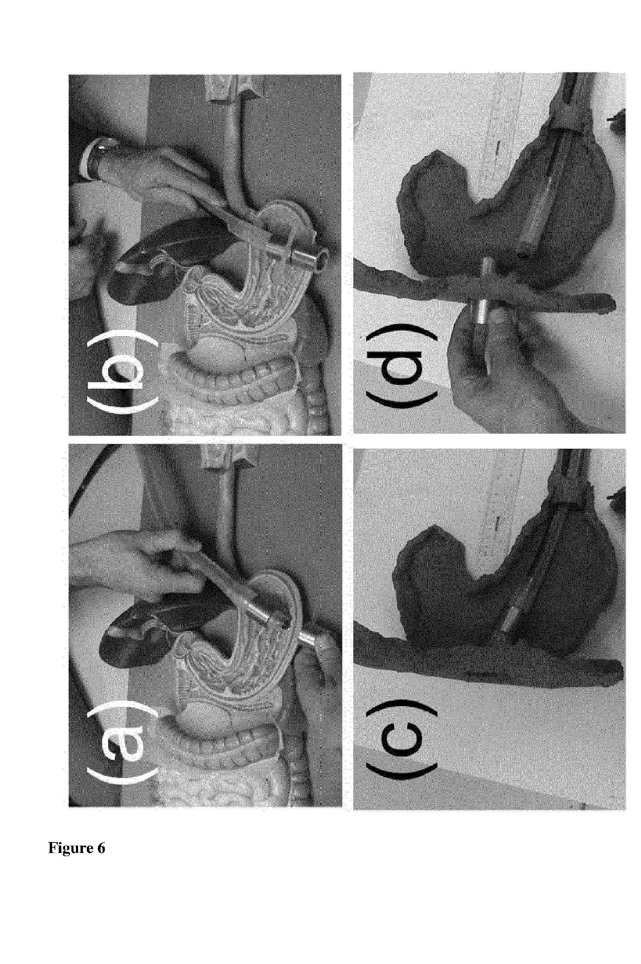

Figure 6 illustrates a demonstration of a procedure using the magnetic coupling PEG tube of the present invention in a benchtop anatomical model for (a) endoscopic navigation impairment and (b) magnetic coupling before testing in the scaled modelling clay gastric model for (c) navigation and (d) transgastric magnetic coupling.

**Detailed Description of the Drawings**

Currently, the technique most commonly used for PEG tube placement is the so-called 'pull' technique, outlined in Figure 1. An endoscope is introduced into the patient's stomach and a combination of trans-illumination (*i.e.*, shining the endoscopic lamp across the gastric and abdominal walls) and finger pressure is used to determine the site of closest contact (Figure 1(a)). The so-called 'safe tract' method involves insertion of a syringe of local anaesthetic which is blindly guided across the abdominal and gastric walls at the site of transillumination (Figure 1(b)). The site is catheterised and an endoscopic snare (introduced via the endoscope's instrument channel) is used to lasso a guidewire which is pushed through the catheterised abdominal and gastric walls (Figure 1(c)). Removing the endoscope leaves the guidewire extending across the abdominal and gastric walls and out of the patient's mouth. The guidewire then serves as the tram-line for the oral introduction of the feeding tube in advance of reintroducing the endoscope for inspection. The distal end of the feeding tube usually has a round bumper to prevent its escape through the gastrotomy (Figure 1(d, e)). The procedure ends when the portion of inserted feeding tube outside the abdominal wall is snipped, the nutrition sack is attached and the endoscope is removed. The external t-bar shown in Figure 1(d) sits above the skin and is designed to stop excessive tension and "buried ring syndrome" where the gastric wall grows over the tube thereby causing obstruction.

One of the advantages of the present invention is to augment the 'safe tract' approach shown in Figures 1(a) and (b) with a simple mechanism of temporary magnetic coupling across the abdominal and gastric walls, as shown in Figure 2. The magnetic coupling of two rings, one on the abdominal wall (external to the patient) and one on the gastric wall (endoscopically delivered) is the second step (*i.e.,* coming between Figures 1(a) and 1(b)) in a slightly modified PEG technique. However, once the coupling is in place, one of the advantageous results is that the relative positions of gastric and abdominal walls are fixed and sandwiched in place at a known minimum separation. A further advantage is that coupling only takes place at a separation less than a critical maximum which can be used as a check for inadvertent sandwiching of organs like the colon between the gastric and abdominal walls during the procedure.

*Materials and Methods*

The critical component of the present invention is the coupling of the two magnetic rings at a known and predictable distance of separation. To investigate, this dependence, the usual magnetic charge model was used to simulate magnetic mating of two permanently magnetised concentric rings. Following the usual Coulombic Law formulation, the force vector exerted by 'magnetic charge', $q_{m1}$, on 'magnetic charge', $q_{m2}$ is given by (1) where $\mu_0$ is the magnetic permeability of free space ($4\pi \times 10^{-7}$ H/m) and $\mathbf{r}_{12}$ is the displacement vector between $q_{m1}$ and $q_{m2}$.

$$\mathbf{F}_{12} = \mu_0\, q_{m1}\, q_{m2} / (4\pi\, \mathbf{r}_{12}^{\,2}) \qquad (1)$$

One of the principal challenges in magnetic coupling is the inverse square roll-off in the force of attraction between magnetic components. The coupling forces between concentric mating magnetic rings were simulated using the open-source *Radia* (ESRF, France) add-on to *Mathematica 7* (*Wolfram Corp.,* Champaign, Illinois). The magnetic force is found by discretisation of all the magnetic surfaces and integration of (1) over the nearby surfaces which are subject to the field (*i.e.,* the adjacent ring). The simulated results, as shown in Figure 3, demonstrate the familiar inverse square relation between force and separation between the two magnetic rings (ignoring gravitational forces) as well as the force associated with coupling an external magnet to internal steel rings (SR1-SR3) of varying dimensions. Based on the investigations of Figure 3, a coupling capable of magnetic mating across expected stomach wall thickness of 3-4 cm was designed. Two permanent magnetic N52 grade neodymium-iron-boron (NdFeB) rings were purchased (26mm OD, 18mm ID and 25mm H) from HKCM Engineering, Germany, for subsequent testing (Figure 4(a)). In addition, a number of mild steel rings (EN3B grade mild steel) were fabricated of various wall thicknesses and lengths (Figure 4 (b)). Because EN3B grade steel has significant paramagnetic properties (*i.e.,* it behaves magnetically in the presence of a magnetic field source such as a permanent magnet with magnetic susceptibility, $\chi \approx 800$), the use of mild steel rings for use as the gastric wall magnet in Figure 2 were also investigated.

The most significant challenge in implementing a magnetic coupling across the gastric and abdominal walls was the placement of the gastric wall magnet within the patient's stomach without any incision. Since the PEG procedure already involves the oral introduction of an endoscope, an endoscope was used as the vehicle to carry the gastric magnet into its final position. A number of approaches were considered including introducing the magnet in advance of the endoscope using a magnetised catheter. However, the technique that was found most satisfactory was spearing the ring with the endoscope's shaft with a radially-inflatable ring balloon between the endoscope and magnet, as shown in Figures 5 and 6. This approach had significant advantages; (i) the magnet could be held in position on the endoscope's shaft by inflation of the balloon and released for coupling by deflation; (ii) inflation of the balloon beyond the ring OD during oral insertion limited any possibility of tearing to the oesophageal wall upon introduction of the endoscope; and (iii) the magnet presented no visual impediment to the scope's field of view.

The ring balloon was constructed from a 51Fr (17mm) veterinary endotracheal tube (Jorgensen Laboratories, Colorado) which was chosen to fit snugly over a standard 12mm diameter endoscope (GIF Q20 by Olympus Inc., Japan). The balloon was lure-lock connected to a standard endovascular balloon indeflator (Boston Scientific Corp., Massachuetts) which was used to inflate the balloon to a measureable pressure as shown in Figure 5(c).

*Results*

The magnetic coupling force predicted by Figure 3 was experimentally investigated by compression and separation tests using a Stable Micro Texture Analyser (Godalming, UK). The resultant force-separation characteristic for the various rings are shown as datapoints on Figure 3.

To accurately predict the required force to couple the gastric and abdominal magnets in the presence of the inflated balloon, the axial force required to slide the gastric magnet off the inflated balloon was also investigated as a function of various balloon inflation pressures. This is an important parameter because, in the modified procedure, the magnetic coupling is mainly impeded by the frictional forces between the balloon and gastric magnet, which varies as a function of inflation pressure, and not gravitational force. The coupling distance (*i.e.*, critical distance at which mating occurs) between the various gastric magnets of Figure 3 and the NdFeB abdominal ring magnet is shown in Table 1. These results correspond to the worst-case scenario where gravity acts against the magnetic coupling force. This is not unrealistic in a clinical setting where the PEG placement usually takes place while the patient lies on their back. However, the coupling distance can be significantly increased by increasing surface area on the external magnet and/or the use of an electromagnet or permanent electromagnet. Coupling distances up to and above 10cm are entirely realisable.

Table 1. Coupling Distance to NdFeB Ring

|  | NdFeB | SR1 | SR2 | SR3 |
|---|---|---|---|---|
| Coupling Dist (mm) | 35±3 | 21±2 | 19±4 | 18±3 |

The modified PEG procedure including magnetic coupling was experimentally demonstrated in a benchtop test using a simplified anatomical model, as shown in Figure 6(a) and (b). The magnetic coupling mechanism and 'steerability' of the endoscope in the presence of the balloon and ring magnet was then investigated using a gastric model made of modelling clay (Figure 6(c) and (d)). In this investigation, the endoscope was advanced through a 2cm diameter rigid tube (simulating the oesophagus) into the model stomach. The endoscope was then flexed at approximately 30° to the horizontal to provide coupling to the external magnetic ring. Coupling occurred at a separation of 3-4cm. After coupling, the gastric magnet was removed by reinserting the endoscope into the ring, inflating the balloon and removing the external magnetic ring.

In present invention, there is provided a simple mechanism that reduces complications in the placement of gastroenteral feeding tubes. The technique relies on the temporary magnetic coupling of two rings, one in the stomach (which is endoscopically delivered) and a second external to the patient. As indicated in Figure 3, the coupling compression forces are highly predictable. Also, depending upon the coupling ring materials, it has been shown in Table 1 that the distance within which coupling occurs can be predicted. Based on expected gastric/abdominal wall separation of 3-4cm and the results of Table 1, two N52 NdFeB rings provide successful coupling. This modification represents a significant advantage over current approaches where there is no knowledge of gastric to abdominal wall separation distance.

The balloon is an excellent means to maintain the gastric ring magnet in position until coupling is needed. To ensure that the manoeuvrability of the endoscope is unimpeded by the balloon, the balloon is designed and constructed with minimal inner wall thickness. The use of a bronchoscope (6-8mm OD) also permits the use of a balloon with minimal inner wall thickness. A second refinement involves the integration of a visual confirmation of mating (*e.g.*, a light-emitting diode or buzzer which turns on upon coupling) attached to the external magnetic ring unit. The external magnet or ring unit can be replaced with an electromagnet. The use of an electromagnet provides a further advantage in that the external magnet cannot be accidently uncoupled from the internal gastric magnet.

The present invention might also be used for positioning of surgical instruments in minimally-invasive procedures such as natural orifice surgery and single-site laparoscopy, where endoscopic instruments are tethered to the gastric wall during procedures by means of gastric to abdominal wall magnetic coupling (*e.g.*, a magnetic camera positioning system).

Furthermore, the magnetic coupling mechanism of the present invention can also be used for PEC procedures where a PEC tube is inserted in a similar way as a PEG tube and connected to a colostomy drainage bag.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

**Claims**

1. A kit suitable for performing a surgical procedure involving making a percutaneous incision and placement of a tube within the percutaneous incision, the kit comprising a tube, and an internal magnet capable of coupling with an external magnet across a gastrointestinal and abdominal wall, the internal magnet including a through-hole dimensioned for receipt of the tube, wherein the through-hole of the internal magnet is capable of aligning with a through-hole of the external magnet upon coupling of the magnets to provide a guide path for passage of the tube.

2. A kit as claimed in Claim 1 and including an external magnet capable of coupling together with the internal magnet across a gastrointestinal and abdominal wall, each magnet including a through-hole dimensioned for receipt of the tube.

3. A kit as claimed in Claim 1 and 2, which is suitable for performing (a) a percutaneous endoscopic gastrostomy, wherein the internal and external magnets are capable of coupling across a gastric and abdominal wall or (b) a percutaneous endoscopic jejunoostomy, wherein the internal and external magnets are capable of coupling across an intestinal and abdominal wall.

4. A kit as claimed in Claim 1, 2 or 3 in which the or each magnet is a ring-shaped magnet.

5. A kit as claimed in any preceding Claims and including a scoping device suitable for positioning the internal magnet inside the gastrointestinal tract and having a distal end, a proximal end, and means for engaging the internal magnet disposed on the distal end.

6. A kit as claimed in Claim 5 in which the means for engaging the internalt magnet comprises an inflatable balloon, wherein inflation of the balloon secures the magnet at the distal end of the device and deflation of the balloon releases the magnet from the distal end of the device.

7. A kit as claimed in any preceding Claim in which the external magnet includes an indicator for confirming when the two magnets have coupled together.

8. A kit as claimed in Claim 6 in which the indicator is an LED light or an audible buzzer.

9. A kit as claimed in any preceding Claim in which the or each magnet is selected from the group comprising neodymium-iron-boron, stainless steel, iron, cobalt, and nickel.

10. A kit as claimed in any preceding Claim further including one, more, or all of a syringe, a cannula, a retrieval snare, a bolus adapter, and a retention ring.

11. An assembled magnetic coupling suitable for assisting placement of a percutaneous tube and comprising an internal magnet disposed inside the gastrointestinal tract of an individual and bearing against an inside of the wall of the gastrointestinal tract, magnetically coupled to an external magnet disposed externally against the abdomen of the individual, each magnet comprising a through-hole dimensioned for receipt of the percutaneous tube, wherein the through-holes are aligned to provide a path for passage of the percutaneous tube through the gastrointestinal and abdominal walls.

12. An assembled magnetic coupling of Claim 11 suitable for assisting placement of: (a) a percutaneous endoscopic gastrostomy feeding tube, in which the internal magnet is disposed inside the stomach of the individual and bears against an inside of the stomach wall, wherein the through-holes are aligned to provide a path for passage of the percutaneous feeding tube through the gastrointestinal; or (b) a percutaneous endoscopic jejunostomy feeding tube, in which the internal magnet is disposed inside the intestine of the individual and bears against an inside of the jejunal wall, wherein the through-holes are aligned to provide a path for passage of the percutaneous feeding tube through the gastrointestinal and jejunal walls.

**13.** An assembled magnetic coupling of Claim 12 in which the external magnetic is an electromagnet, optionally selected from a reusable electromagnet or a permanent electromagnet.

**14.** A scoping device suitable for being inserted into the body and relaying images from inside the body to a viewing device located outside the body, the device having a distal end and a proximal end, **characterised in that** the distal end of the device comprises an inflatable balloon, wherein inflation and deflation of the balloon can be controlled from the proximal end of the device.

**15.** A kit suitable for providing a percutaneous incision useful for receipt of a gastroenteral feeding tube or a colostomy tube, the kit comprising a scoping device of Claim 14, and at least one magnet having a through hole, wherein the through-hole is dimensioned for receipt of the inflatable balloon when deflated but not when inflated.

**Figure 1**

**Figure 2**

**Figure 3**

**Figure 4**

Figure 5

Figure 6

Europäisches
Patentamt

European
Patent Office

Office européen
des brevets

# EUROPEAN SEARCH REPORT

Application Number

EP 11 19 3758

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/097124 A2 (US ENDOSCOPY GROUP INC [US]; GINSBERG GREGORY G [US]) 27 November 2003 (2003-11-27) * pages 5-8; figures 2,4,6 * | 1-5,9,10 | INV. A61B17/00 A61B17/34 |
| X | US 2009/318854 A1 (BAILEY F KRISTEN [US]) 24 December 2009 (2009-12-24) * paragraphs [0002], [0023], [0024], [0030]; figures 1,6 * | 1-5,9,10 | |
| X | WO 2011/143174 A1 (AGUIRRE ANDRES F [US]; DUCHARME RICHARD W [US]) 17 November 2011 (2011-11-17) * paragraphs [0017], [0024], [0029]; figures 1,5 * | 1-4,9,10 | |
| X | US 2010/179510 A1 (FOX WILLIAM D [US] ET AL) 15 July 2010 (2010-07-15) * paragraphs [0044], [0045], [0051]; figures 1,4A,4C,11B * | 1-6,9,10 | |
| | | | TECHNICAL FIELDS SEARCHED (IPC) A61B A61J |

The present search report has been drawn up for all claims

| Place of search | Date of completion of the search | Examiner |
|---|---|---|
| Munich | 26 April 2012 | Assion, Jean-Charles |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

Application Number

EP 11 19 3758

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-13

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 11 19 3758

The Search Division considers that the present European patent application does not comply with the
requirements of unity of invention and relates to several inventions or groups of inventions, namely:

```
1. claims: 1-13

        kit comprising a tube and an internal magnet
                          ---

2. claims: 14, 15

        scoping device comprising an inflatable balloon
                          ---
```

## ANNEX TO THE EUROPEAN SEARCH REPORT
## ON EUROPEAN PATENT APPLICATION NO.

EP 11 19 3758

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2012

| Patent document cited in search report | | Publication date | Patent family member(s) | | Publication date |
|---|---|---|---|---|---|
| WO 03097124 | A2 | 27-11-2003 | AU 2003230419 A1 | | 02-12-2003 |
| | | | US 2005177174 A1 | | 11-08-2005 |
| | | | WO 03097124 A2 | | 27-11-2003 |
| US 2009318854 | A1 | 24-12-2009 | NONE | | |
| WO 2011143174 | A1 | 17-11-2011 | US 2011288534 A1 | | 24-11-2011 |
| | | | WO 2011143174 A1 | | 17-11-2011 |
| US 2010179510 | A1 | 15-07-2010 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PONSKY.** Percutaneous endoscopic gastrostomy. *Journal of Gastrointestinal Surgery,* 2004, vol. 8 (7), 901-904